# EUROPEAN PATENT APPLICATION

(11) **EP 4 585 169 A1**
(43) Date of publication of application: **16.07.2025**
(21) Application number: 24305077.0
(22) Date of filing: 11.01.2024
(51) Int. Cl.: A61B 17/16, A61B 17/88, A61B 90/00

(54) **AN INSTRUMENTATION SYSTEM FOR PERCUTANEOUSLY FIXING A HEADLESS BONE SCREW INTO A PATIENT'S BONE**

(71) Applicant: FH ORTHO, 68990 Heimsbrunn (FR); Fournitures Hospitalieres Industrie, 29000 Quimper (FR)
(72) Inventor: CAROFF, Julien, 29000 Quimper (FR)
(74) Representative: Germain Maureau

(57) **Abstract**

The instrumentation system (2) includes a first tool (5) including a first longitudinal body (6) and first graduations (9) provided on an external surface of the first longitudinal body (6) and being defined with respect to a first reference point (11) located at the distal end of the first longitudinal body (6); and a second tool (12) including a second longitudinal body (13) having a distal end part (13.1) provided with a rotational drive member configured to cooperate with a corresponding rotational drive element provided on a headless bone screw (3), the second tool (12) including second graduations (17) provided on an external surface of the second longitudinal body (13) and being defined with respect to a second reference point (18) provided on the distal end part (13.1) and configured to be located at a proximal end face (3.1) of the headless bone screw (3) when the rotational drive member cooperates with the corresponding rotational drive element.

## Description

### Field of the invention

The present invention relates to instrumentation system for percutaneously fixing a headless bone screw into a patient's bone

### Background art

Different surgical techniques exist to consolidate a complex fracture that require moving fractured bone fragments.

One of said surgical techniques consists in inserting osteosynthesis bone screws or so-called "compression" osteosynthesis screws into the fractured bone fragments so as to secure the latter to the main part of the bone in order to allow natural bone consolidation of the fracture site. Such screws are generally placed percutaneously, and only require a small cutaneous incision.

However, an osteosynthesis bone screw may be provided with a screw head configured to bear against the cortical bone surface of the respective bone fragment, and such a screw head may generate a painful friction on the patient's muscles.

In order to avoid generation of painful friction, it is known to secure fractured bone fragments using osteosynthesis headless screws. However, due to the small size of the cutaneous incision made in the patient's skin and the presence of soft tissue, it is difficult for the surgeon to accurately position an osteosynthesis headless screw with respect to the cortical bone surface, and particularly to implant the osteosynthesis headless screw at the right depth in the bone. Yet, if the osteosynthesis headless screw is implanted too deep in the bone, the osteosynthesis headless screw may migrate with respect to the respective bone fragment leading to possible movements of the latter and to a poor bone consolidation, and if the osteosynthesis headless screw is implanted not deep enough in the bone, tissues irritation and pain or discomfort may be generated.

In addition, following an accidental rupture of an anterior or posterior cruciate ligament of a knee joint, it is known to replace the ruptured anterior or posterior cruciate ligament with a ligament transplant and to secure ends of said ligament transplant respectively to the respective femur and the respective tibia via fastening devices.

A known ligament transplant fastening device consists of a headless interference screw configured to anchor a tension strip, attached to an end of the ligament transplant, in a cylindrical bone tunnel, by jamming the tension strip between the threads of the interference screw and the inner wall of the bone tunnel in which a screw thread has been previously made.

However, due to the small size of the cutaneous incision made in the patient's skin and the presence of soft tissue, it is difficult for the surgeon to accurately position a headless interference screw with respect to the cortical bone surface, and particularly to implant the headless interference screw at the right depth in the bone. Yet, if the headless interference screw is implanted too deep in the bone, the headless interference screw may migrate leading to a chess of the ligament reconstruction, and if the headless interference screw is implanted not deep enough in the bone, tissues irritation and pain or discomfort may be generated.

### Summary of the invention

It is an object of the present invention to overcome the above-mentioned drawbacks from the prior art solutions and provide an instrumentation system for percutaneously fixing a headless bone screw into a patient's bone which is simple and ergonomic, while reducing surgery time and patient recovery time and while limiting the risks of subsequent pain for the patient.

According to the present invention, there is provided an instrumentation system for percutaneously fixing a headless bone screw into a bone of a patient, the instrumentation system including at least:
- a first tool including a first longitudinal body provided with a first axial guide channel extending over at least a part of the length of the first longitudinal body and having a first distal channel end emerging in a distal end of the first longitudinal body, the first axial guide channel being configured to receive and slidingly cooperate with a guide pin implanted in the bone of the patient and protruding from a cutaneous incision made in the skin of the patient,
- a second tool including a second longitudinal body provided with a second axial guide channel extending over at least a part of the length of the second longitudinal body and having a second distal channel end emerging in a distal end of the second longitudinal body, the second axial guide channel being configured to receive and slidingly cooperate with the guide pin implanted in the bone of the patient, the second longitudinal body further including a distal end part provided with a rotational drive member configured to cooperate with a corresponding rotational drive element provided on the headless bone screw and to transmit a screwing torque to the headless bone screw,
characterized in that the first tool includes a first series of first graduations which are longitudinally offset and provided on an external surface of the first longitudinal body, the first graduations being defined with respect to a first reference point located at the distal end of the first longitudinal body, the first graduations being configured to enable a surgeon to determine, directly by eye, a reference graduation representative of an axial distance between a cortical surface (i.e. an outer surface) of the bone and an outer surface of the patient's skin (i.e. to determine, directly or indirectly, a soft tissues thickness) at the cutaneous incision when the distal end of the first longitudinal body, guided by the guide pin received in the first axial guide channel, abuts against the cortical surface of the bone, and in that the second tool includes a second series of second graduations which are longitudinally offset and provided on an external surface of the second longitudinal body, the second graduations being defined with respect to a second reference point provided on the distal end part of the second longitudinal body and configured to be located at a proximal end face of the headless bone screw when the rotational drive member provided on the second longitudinal body cooperates with the corresponding rotational drive element provided on the headless bone screw, each of the second graduations corresponding to a respective first graduation of the first graduations.

Such a configuration of the instrumentation system according to the present invention allows a surgeon to easily determine, using the first tool, a reference graduation representative of an axial distance between a cortical surface of the bone and an outer surface of the patient's skin at the cutaneous incision, and then to accurately position a headless bone screw in the bone, using the second tool, by stopping the screwing of the headless bone screw when a second graduation, provided on the second tool and corresponding to the determined reference graduation, is located at the level of the outer surface of the patient's skin.

Particularly, the instrumentation system according to the present invention allows a surgeon to accurately position the headless bone screw (i.e. at the right depth) such that the proximal end face of the headless bone screw is flush with the cortical surface of the bone, which avoids any subsequent migration of the headless bone screw and any generation of pain or discomfort for the patient.

The instrumentation system may also include one or more of the following features, taken alone or in combination.

According to an embodiment of the invention, the first tool is a reamer.

According to an embodiment of the invention, the first tool is configured to drill, in the bone, a bone hole or a bone tunnel configured to receive the headless bone screw.

According to an embodiment of the invention, the first tool is configured to magnify, at least in a bone area in which the headless bone screw is to be fixed, a diameter of a bone hole or a bone tunnel drilled in the bone and configured to receive the headless bone screw.

According to an embodiment of the invention, the second tool is a screwing tool, such as a screwdriver.

According to an embodiment of the invention, the rotational drive member provided on the second longitudinal body is a male rotational drive member and the corresponding rotational drive element provided on the headless bone screw is a female rotational drive element, such as a hexagon socket recess or a hexalobular recess.

According to an embodiment of the invention, the rotational drive member has a hexagonal cross-section, a triangular cross-section or a hexalobular cross-section.

According to an embodiment of the invention, each of the first graduations corresponds to a respective axial distance from the first reference point, and each of the second graduations corresponds to a respective axial distance from the second reference point.

According to an embodiment of the invention, the first axial guide channel extends over the entire length of the first longitudinal body and emerges in a proximal end of the first longitudinal body, and the second axial guide channel extends over the entire length of the second longitudinal body and emerges in a proximal end of the second longitudinal body.

According to an embodiment of the invention, the instrumentation system further includes a third tool including a third longitudinal body provided with a third axial guide channel extending over at least a part of the length of the third longitudinal body and having a third distal channel end emerging in a distal end of the third longitudinal body, the third axial guide channel being configured to receive and slidingly cooperate with the guide pin implanted in the bone of the patient, the third longitudinal body further including a distal tapping part configured to create a screw thread (i.e. a tapping) into a pre-drilled bone hole or bone tunnel configured to receive the headless bone screw, the third tool including a third series of third graduations which are longitudinally offset and provided on an external surface of the third longitudinal body, the third graduations being defined with respect to a third reference point provided on the distal tapping part of the third longitudinal body, each of the third graduations corresponding to a respective first graduation of the first graduations.

According to an embodiment of the invention, the third tool is a tap, also named tapper.

According to an embodiment of the invention, each of the first and second graduations is formed by laser marking.

The present invention also relates to an instrumentation kit including an instrumentation system according to the invention, and at least one headless bone screw.

According to an embodiment of the invention, the headless bone screw is configured to anchor a tension strip, attached to a ligament graft, in a bone tunnel, and particularly to immobilizing the tension strip against an inner wall of a bone tunnel in which a screw thread has been previously made.

According to an embodiment of the invention, the headless bone screw is a percutaneous osteosynthesis screw.

According to an embodiment of the invention, the headless bone screw includes a screw body and a guide channel extending axially over the entire length of the screw body and having a distal channel end emerging in a distal end of the screw body and a proximal channel end emerging in a proximal end of the screw body.

According to an embodiment of the invention, the headless bone screw is an orthopaedic screw.

According to an embodiment of the invention, the first graduations are spaced 5 mm apart axially, and the second graduations are spaced 5 mm apart axially.

The present invention also relates to a method for fixing a headless bone screw in a bone, including:
- introducing a guide pin in the bone through a cutaneous incision made in a patient's skin,
- providing an instrumentation system according to the present invention,
- engaging the guide pin in the first axial channel of the first tool and displacing the first tool along the guide pin and towards the bone until the distal end of the first longitudinal body abuts against the cortical surface of the bone,
- reading the first graduation located at the outer surface of the patient's skin when the distal end of the first longitudinal body abuts against the cortical surface of the bone,
- screwing the headless bone screw into the bone, and for example in a bone hole or a bone tunnel drilled in the bone, using the second tool,
- stopping the screwing step when a second graduation, provided on the second longitudinal body and corresponding to the first graduation read during the reading step, is located at the outer surface of the patient's skin, and
- disengaging the second tool from the headless bone screw.

According to an embodiment of the invention, the method further includes, prior to the screwing step, drilling, in the bone and using the first tool, a bone hole or a bone tunnel configured to receive the headless bone screw.

According to an embodiment of the invention, the method further includes, prior to the screwing step, magnifying, at least in a bone area in which the headless bone screw is to be fixed, a diameter of a bone hole or bone tunnel, pre-drilled in the bone and configured to receive the headless bone screw, using the first tool.

According to an embodiment of the invention, the method further includes creating a screw thread (i.e. a tapping) into a pre-drilled bone hole or bone tunnel configured to receive the headless bone screw, using the third tool, and stopping the creating step when a third graduation, provided on the third longitudinal body and corresponding to the first graduation read during the reading step, is located at the outer surface of the patient's skin.

### Brief description of the drawings

The following detailed description of an embodiment of the invention is better understood when read in conjunction with the appended drawings being understood, however, that the invention is not limited to the specific embodiment disclosed.
Figure 1 is a view showing successive steps (from the left to the right) of a method for fixing a headless bone screw into a patient's bone using an instrumentation system according to a first embodiment of the invention.
Figure 2 is a longitudinal cross-section view showing the successive steps of figure 1.
Figure 3 is a perspective view of a first tool of the instrumentation system of figure 1.
Figure 4 is a perspective view of a second tool of the instrumentation system of figure 1.
Figure 5 is a view showing successive steps (from the left to the right) of a method for fixing a headless bone screw into a patient's bone using an instrumentation system according to a second embodiment of the invention.
Figure 6 is a longitudinal cross-section view showing the successive steps of figure 5.

### Description of the preferred embodiment of the invention

In the present document, the terms "distal" and "proximal" are defined with respect to the surgeon.

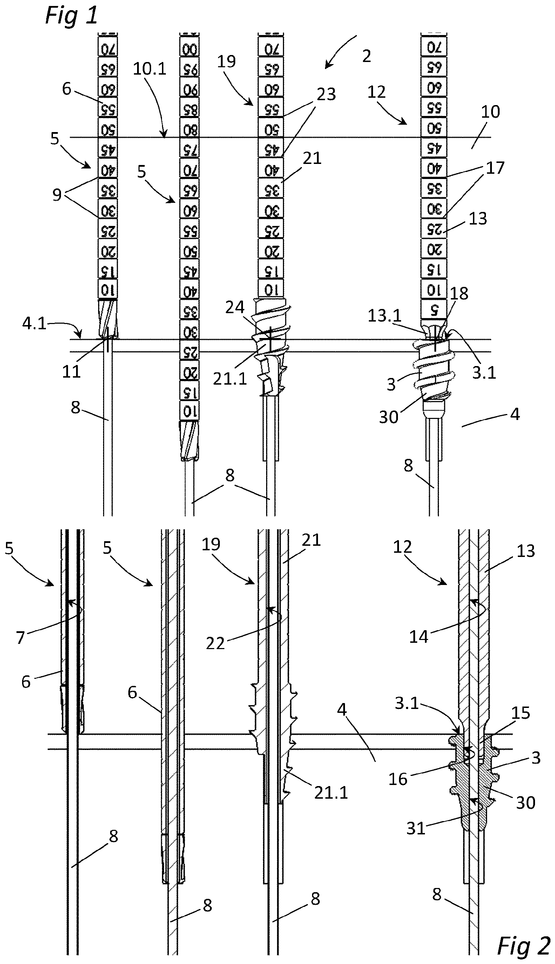

Figures 1 to 4 show an instrumentation system 2, according to a first embodiment of the invention, for percutaneously fixing a headless bone screw 3 into a bone 4 of a patient, and for example in a bone hole or a bone tunnel drilled in said bone 4 and configured to receive the headless bone screw 3.

The instrumentation system 2 includes a first tool 5 including a first longitudinal body 6 provided with a first axial guide channel 7 extending over at least a part of the length, and for example over the entire length, of the first longitudinal body 6. The first axial guide channel 7 has a first distal channel end emerging in a distal end of the first longitudinal body 6. The first axial guide channel 7 is configured to receive and slidingly cooperate with a guide pin 8 implanted in the patient's bone 4 and protruding from a cutaneous incision made in the patient's skin 10.

The first tool 5 further includes a first series of first graduations 9 which are longitudinally offset and provided on an external surface of the first longitudinal body 6. The first graduations 9 are defined with respect to a first reference point 11 located at the distal end of the first longitudinal body 6.

The first graduations 9 are configured to enable a surgeon to determine, directly by eye, a reference graduation representative of an axial distance between a cortical surface 4.1 of the bone 4 and an outer surface 10.1 of the patient's skin 10 (i.e. to determine, directly or indirectly, a soft tissues thickness) at the cutaneous incision when the distal end of the first longitudinal body 6, guided by the guide pin 8 received in the first axial guide channel 7, abuts against the cortical surface 4.1 of the bone 4.

According to the embodiment shown on the drawings, each of the first graduations 9 corresponds to a respective axial distance from the first reference point 11 indicated in mm, and the first graduations 9 are spaced 5 mm apart axially. However, according to an alternative embodiment of the invention, the first graduations 9 may be landmark, for example named A, B, C, D ..., also spaced 5 mm apart axially.

According to the embodiment shown on figures 1 to 4, the first tool 5 is a reamer configured to drill, in the bone 4, a bone hole or a bone tunnel configured to receive the headless bone screw 3. Such a reamer may also be configured to magnify, at least in a bone area in which the headless bone screw 3 is to be fixed, a diameter of a bone hole or a bone tunnel pre-drilled in the bone 4 and configured to receive the headless bone screw 3.

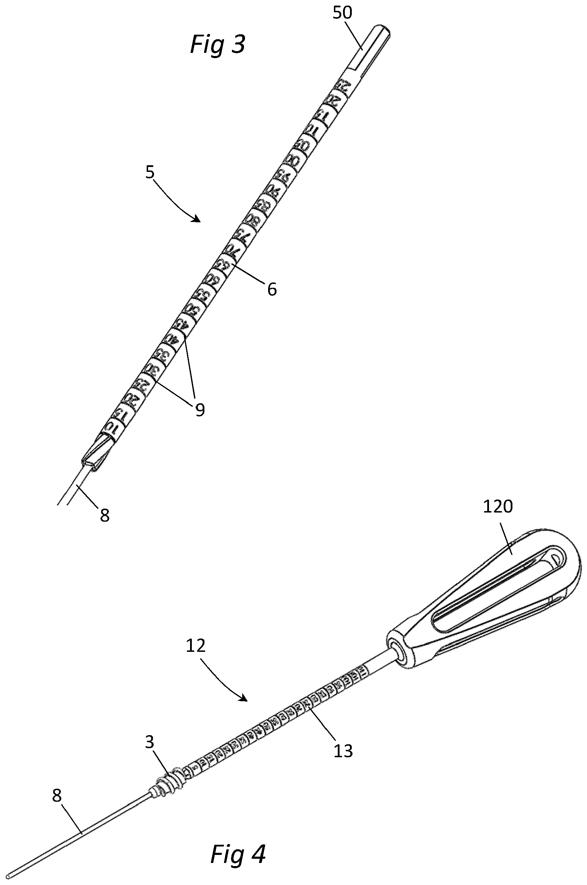

The first tool 5, and for example the first longitudinal body 6, may for example includes (see figure 3) a proximal connecting part 50 configured to be connected to a rotation drive apparatus (not shown on the figures) configured to drive in rotation the first tool 5 about a rotation axis coaxial with a longitudinal central axis of the first longitudinal body 6.

The instrumentation system 2 also includes a second tool 12, such as a screwdriver, including a second longitudinal body 13 provided with a second axial guide channel 14 extending over at least a part of the length, and for example over the entire length, of the second longitudinal body 13. The second axial guide channel 14 has a second distal channel end emerging in a distal end of the second longitudinal body 13. The second axial guide channel 14 is configured to receive and slidingly cooperate with a guide pin 8 implanted in the patient's bone 4.

The second longitudinal body 13 further includes a distal end part 13.1 provided with a rotational drive member 15 configured to cooperate with a corresponding rotational drive element 16 provided on the headless bone screw 3 and to transmit a screwing torque to the headless bone screw 3.

According to the embodiment shown on the drawings, the rotational drive member 15 provided on the second longitudinal body 13 is a male rotational drive member, such as a hexagon bit or triangular bit, and the corresponding rotational drive element 16 provided on the headless bone screw 3 is a female rotational drive element, such as a hexagon socket recess or a triangular socket recess, emerging in a proximal end face 3.1 of the headless bone screw 3.

The second tool 12 further includes a second series of second graduations 17 which are longitudinally offset and provided on an external surface of the second longitudinal body 13. The second graduations 17 are defined with respect to a second reference point 18 provided on the distal end part 13.1 of the second longitudinal body 13 and configured to be located at the proximal end face 3.1 of the headless bone screw 3 when the rotational drive member 15 provided on the second longitudinal body 13 cooperates with the corresponding rotational drive element 16 provided on the headless bone screw 3.

Each of the second graduations 17 corresponds to a respective first graduation 9. Thus, according to the embodiment shown on the drawings, each of the second graduations 17 corresponds to a respective axial distance from the second reference point 18 indicated in mm, and the second graduations 17 are spaced 5 mm apart axially. However, according to an alternative embodiment of the invention, the second graduations 17 may be landmark, for example named A, B, C, D ..., also spaced 5 mm apart axially.

The second tool 12 may for example includes (see figure 4) a proximal handling part 120 secured to a proximal end of the second longitudinal body 13 and configured to be handled by a surgeon in order to drive in rotation the second tool 12 about a rotation axis coaxial with a longitudinal central axis of the second longitudinal body 13.

Advantageously, the instrumentation system 2 further includes a third tool 19, such as a tap, including a third longitudinal body 21 provided with a third axial guide channel 22 extending over at least a part of the length, and for example over the entire length, of the third longitudinal body 21. The third axial guide channel 22 has a third distal channel end emerging in a distal end of the third longitudinal body 21. The third axial guide channel 22 is configured to receive and slidingly cooperate with a guide pin 8 implanted in the patient's bone 4.

The third longitudinal body 21 further includes a distal tapping part 21.1 configured to create a screw thread (i.e. a tapping) into a pre-drilled bone hole or bone tunnel configured to receive the headless bone screw 3.

The third tool 19 also includes a third series of third graduations 23 which are longitudinally offset and provided on an external surface of the third longitudinal body 21. The third graduations 23 are defined with respect to a third reference point 24 provided on the distal tapping part 21.1 of the third longitudinal body 21.

Each of the third graduations 23 corresponds to a respective first graduation 9. Thus, according to the embodiment shown on the drawings, each of the third graduations 23 corresponds to a respective axial distance from the third reference point 24 indicated in mm, and the third graduations 23 are spaced 5 mm apart axially. However, according to an alternative embodiment of the invention, the third graduations 23 may be landmark, for example named A, B, C, D ..., also spaced 5 mm apart axially.

According to an embodiment of the invention, each of the first graduations 9, second graduations 17 and third graduations 23 is formed by laser marking.

The third tool 19, and for example the third longitudinal body 21, may for example includes a proximal connecting part (not shown on the figures) configured to be connected to a rotation drive apparatus configured to drive in rotation the third tool 19 about a rotation axis coaxial with a longitudinal central axis of the third longitudinal body 6.

According to the embodiment shown on figures 1 to 4, the headless bone screw 3 includes a screw body 30, and a guide channel 31 extending axially over the entire length of the screw body 30 and having a distal channel end emerging in a distal end of the screw body 30 and a proximal channel end emerging in the corresponding rotational drive element 16. Advantageously, the guide channel 31 is configured to receive and slidingly cooperate with the guide pin 8 implanted in the patient's bone 4. Such a configuration of the headless bone screw 3 ensure a proper screwing of the in the bone 4, and particularly avoids that the headless bone screw 3 is offset from the central axis of the bone hone or bone tunnel configured to receive the headless bone screw 3.

The headless bone screw 3 may be an orthopaedic screw, and may be configured to anchor a tension strip, attached to a ligament graft, in a bone tunnel, and particularly to immobilizing the tension strip against an inner wall of the bone tunnel in which a screw thread has been previously made. According to such an embodiment of the invention, the headless bone screw 3 may be made in PEEK.

A method for fixing a headless bone screw 3 in a bone 4 with the instrumentation system 2 according to the present invention is disclosed hereafter.

The method includes the following steps:
- introducing a guide pin 8 in the bone 4 through a cutaneous incision made in a patient's skin 10,
- providing an instrumentation system 2 according to the present invention,
- engaging the guide pin 8 in the first axial channel of the first tool 5 and displacing the first tool 5 along the guide pin 8 and towards the bone 4 until the distal end of the first longitudinal body 6 abuts against the cortical surface 4.1 of the bone 4,
- reading the first graduation 9 located at the outer surface 10.1 of the patient's skin 10 when the distal end of the first longitudinal body abuts against the cortical surface 4.1 of the bone,
- screwing the headless bone screw 3 into the bone 4, and for example in a bone hole or a bone tunnel drilled in the bone 4, using the second tool 12,
- stopping the screwing step when a second graduation 17, provided on the second longitudinal body 13 and corresponding to the first graduation 9 read during the reading step, is located at the level of the outer surface 10.1 of the patient's skin 10, and
- disengaging the second tool 12 from the headless bone screw 3.

Such a configuration of the instrumentation system 2 according to the present invention allows a surgeon to easily determine, using the first tool 5, a reference graduation representative of an axial distance between the cortical surface 4.1 of the bone 4 and the outer surface 10.1 of the patient's skin 10 at the cutaneous incision, and then to accurately position the headless bone screw 3 in the bone 4, using the second tool 12, by stopping the screwing of the headless bone screw 3 when a second graduation 17, provided on the second tool 12 and corresponding to the determined reference graduation, is located at the level of the outer surface 10.1 of the patient's skin 10. Particularly, the instrumentation system 2 according to the present invention allows a surgeon to accurately position the headless bone screw 3 such that the proximal end face 3.1 of the headless bone screw 3 is flush with the cortical surface 4.1 of the bone 4, which avoids any subsequent migration of the headless bone screw 3 and any generation of pain or discomfort for the patient.

According to an embodiment of the invention, the method further includes, after the reading step and prior to the screwing step, the following steps:
- drilling, in the bone 4 and using the first tool 5, a bone hole or a bone tunnel configured to receive the headless bone screw 3,
- creating a screw thread (i.e. a tapping) into the bone hole or bone tunnel configured to receive the headless bone screw 3, using the third tool 19, and
- stopping the creating step when a third graduation 23, provided on the third longitudinal body 21 and corresponding to the first graduation 9 read during the reading step, is located at the level of the outer surface 10.1 of the patient's skin 10.

Said additional steps are particularly required when the headless bone screw 3 is used to anchor a tension strip, attached to a ligament graft, in a bone tunnel.

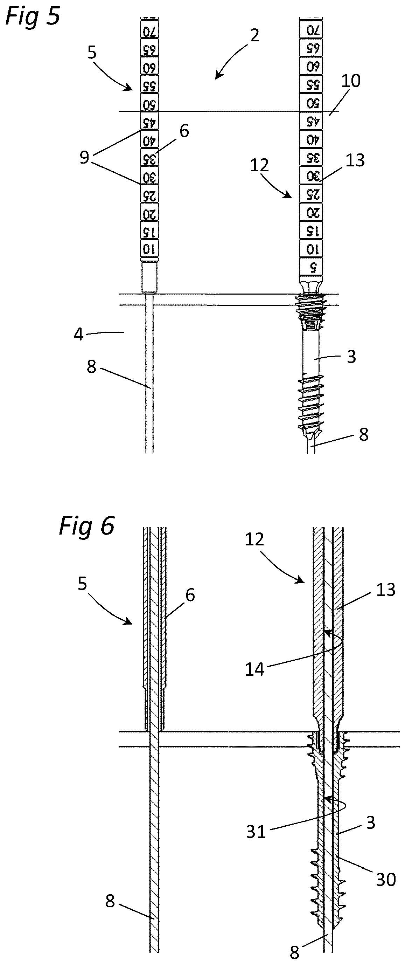

Figures 5 and 6 show an instrumentation system 2 according to a second embodiment of the invention which differs from the first embodiment shown on figures 1 to 4 essentially in that the instrumentation system 2 only includes the first tool 5 and the second tool 12, and in that the tool 5 is a simple measuring tool configured only to enable the surgeon to determine the reference graduation.

According to said embodiment of the invention, the headless bone screw 3 may be a percutaneous osteosynthesis screw and may be configured to compress bone fragments. Thus, the headless bone screw 3 may include a proximal part provided with an outer proximal thread, a distal part provided with a distal thread having a screw pitch different from the one of the outer proximal thread, and an intermediate part located between the proximal part and the distal and being smooth, i.e. devoid of an outer thread. According to such an embodiment of the invention, the headless bone screw 3 may be made in titanium.

Advantageously, a distal end part of the screw body 30 may include at least one tooth configured to drill a bone hole in the bone 4 during the screwing of the headless bone screw 3 in the bone. When using such a headless bone screw 3, it is not required to pre-drill a bone hone configured to receive the headless bone screw 3 before screwing the headless bone screw 3 in the bone 4.

Of course, the invention is not restricted to the embodiments described above by way of nonlimiting examples, but on the contrary it encompasses all embodiments thereof.

## Claims

1. An instrumentation system (2) for percutaneously fixing a headless bone screw (3) into a bone (4) of a patient, the instrumentation system (2) including at least:
- a first tool (5) including a first longitudinal body (6) provided with a first axial guide channel (7) extending over at least a part of the length of the first longitudinal body (6) and having a first distal channel end emerging in a distal end of the first longitudinal body (6), the first axial guide channel (7) being configured to receive and slidingly cooperate with a guide pin (8) implanted in the bone (4) of the patient and protruding from a cutaneous incision made in the skin (10) of the patient,
- a second tool (12) including a second longitudinal body (13) provided with a second axial guide channel (14) extending over at least a part of the length of the second longitudinal body (13) and having a second distal channel end emerging in a distal end of the second longitudinal body (13), the second axial guide channel (14) being configured to receive and slidingly cooperate with the guide pin (8) implanted in the bone (4) of the patient, the second longitudinal body (13) further including a distal end part (13.1) provided with a rotational drive member (15) configured to cooperate with a corresponding rotational drive element (16) provided on the headless bone screw (3) and to transmit a screwing torque to the headless bone screw (3),
**characterized in that** the first tool (5) includes a first series of first graduations (9) which are longitudinally offset and provided on an external surface of the first longitudinal body (6), the first graduations (9) being defined with respect to a first reference point (11) located at the distal end of the first longitudinal body (6), the first graduations (9) being configured to enable a surgeon to determine a reference graduation representative of an axial distance between a cortical surface (4.1) of the bone (4) and an outer surface (10.1) of the patient's skin (10) at the cutaneous incision when the distal end of the first longitudinal body (6), guided by the guide pin (8) received in the first axial guide channel (7), abuts against the cortical surface (4.1) of the bone (4), **and in that** the second tool (12) includes a second series of second graduations (17) which are longitudinally offset and provided on an external surface of the second longitudinal body (13), the second graduations (17) being defined with respect to a second reference point (18) provided on the distal end part (13.1) of the second longitudinal body (13) and configured to be located at a proximal end face (3.1) of the headless bone screw (3) when the rotational drive member (15) provided on the second longitudinal body (13) cooperates with the corresponding rotational drive element (16) provided on the headless bone screw (3), each of the second graduations (17) corresponding to a respective first graduation (9) of the first graduations (9).

2. The instrumentation system (2) according to claim 1, wherein the first tool (5) is a reamer.

3. The instrumentation system (2) according to claim 1 or 2, wherein the second tool (12) is a screwing tool.

4. The instrumentation system (2) according to any one of claims 1 to 3, wherein the rotational drive member (15) provided on the second longitudinal body (13) is a male rotational drive member and the corresponding rotational drive element (16) provided on the headless bone screw (3) is a female rotational drive element.

5. The instrumentation system (2) according to any one of claims 1 to 4, wherein each of the first graduations (9) corresponds to a respective axial distance from the first reference point (11), and each of the second graduations (17) corresponds to a respective axial distance from the second reference point (18).

6. The instrumentation system (2) according to any one of claims 1 to 5, further including a third tool (19) including a third longitudinal body (21) provided with a third axial guide channel (22) extending over at least a part of the length of the third longitudinal body (21) and having a third distal channel end emerging in a distal end of the third longitudinal body (21), the third axial guide channel (22) being configured to receive and slidingly cooperate with the guide pin (8) implanted in the bone (4) of the patient, the third longitudinal body (21) further including a distal tapping part (21.1) configured to create a screw thread into a pre-drilled bone hole or bone tunnel configured to receive the headless bone screw (3), the third tool (19) including a third series of third graduations (23) which are longitudinally offset and provided on an external surface of the third longitudinal body (21), the third graduations (23) being defined with respect to a third reference point (24) provided on the distal tapping part (21.1) of the third longitudinal body (21), each of the third graduations (23) corresponding to a respective first graduation (9) of the first graduations (9).

7. An instrumentation kit including an instrumentation system (2) according to any one of claims 1 to 6, and at least one headless bone screw (3).

8. The instrumentation kit according to claim 7, wherein the headless bone screw (3) is configured to anchor a tension strip, attached to a ligament graft, in a bone tunnel.

9. The instrumentation kit according to claim 7 or 8, wherein the headless bone screw (3) includes a screw body (30) and a guide channel (31) extending axially over the entire length of the screw body (30) and having a distal channel end emerging in a distal end of the screw body (30) and a proximal channel end emerging in a proximal end of the screw body (30).

10. A method for fixing a headless bone screw (3) in a bone (4), including:
- introducing a guide pin (8) in the bone (4) through a cutaneous incision made in a patient's skin (10),
- providing an instrumentation system (2) according to any one of claims 1 to 8,
- engaging the guide pin (8) in the first axial channel of the first tool (5) and displacing the first tool (5) along the guide pin (8) and towards the bone (4) until the distal end of the first longitudinal body (6) abuts against the cortical surface (4.1) of the bone (4),
- reading the first graduation (9) located at the outer surface (10.1) of the patient's skin (10) when the distal end of the first longitudinal body (6) abuts against the cortical surface (4.1) of the bone (4),
- screwing the headless bone screw (3) into the bone (4), using the second tool (12),
- stopping the screwing step when a second graduation (17), provided on the second longitudinal body (13) and corresponding to the first graduation (9) read during the reading step, is located at the outer surface (10.1) of the patient's skin (10), and
- disengaging the second tool (12) from the headless bone screw (3).
